# EUROPEAN PATENT SPECIFICATION

(11) **EP 1 662 873 B1**
(45) Date of publication and mention of the grant of the patent: **13.01.2016**
(21) Application number: 04783478.3
(22) Date of filing: 08.09.2004
(51) Int. Cl.: A01N 37/12, A01N 31/06, A01N 31/02

(54) **ANTIMICROBIAL COMPOSITIONS AND METHODS**
ANTIMIKROBIELLE ZUSAMMENSETZUNGHEN UND VERFAHREN
COMPOSITIONS ANTIMICROBIENNES ET PROCEDES

(30) Priority: 09.09.2003 US 501817 P
(43) Date of publication of application: 07.06.2006
(73) Proprietor: 3M Innovative Properties Company, St. Paul, MN 55133-3427 (US)
(72) Inventor: WANG, Danli, 3M Center, Saint Paul, MN 55133-3427 (US); SCHOLZ, Matthew T., 3M Center, Saint Paul, MN 55133-3427 (US); MITRA, Sumita, B. 3M Center, Saint Paul, MN 55133-3427 (US); VELAMAKANNI, Bhaskar, V. 3M Center, Saint Paul, MN 55133-3427 (US)
(74) Representative: Elsy, David
(86) International application number: PCT/US2004/029241
(87) International publication number: WO 2005/022998

(56) References cited:
- EP-A- 0 104 346
- EP-A- 0 547 727
- EP-A- 0 629 347
- WO-A-01/43549
- DE-A1- 19 642 127
- DE-A1-102004 034 691
- FR-A- 2 729 050
- US-A- 4 067 997
- DATABASE BIOSIS [Online] BIOSCIENCES INFORMATION SERVICE, PHILADELPHIA, PA, US; October 2002 (2002-10), WAKABAYASHI HIROYUKI ET AL: "Increased Staphylococcus-killing activity of an antimicrobial peptide, lactoferricin B, with minocycline and monoacylglycerol." XP002316392 Database accession no. PREV200300092914 & BIOSCIENCE BIOTECHNOLOGY AND BIOCHEMISTRY, vol. 66, no. 10, October 2002 (2002-10), pages 2161-2167, ISSN: 0916-8451
- DATABASE WPI Section Ch, Week 199340 Derwent Publications Ltd., London, GB; Class D21, AN 1993-317359 XP002316394 & JP 05 229915 A (KANEBO LTD) 7 September 1993 (1993-09-07)
- DATABASE BIOSIS [Online] BIOSCIENCES INFORMATION SERVICE, PHILADELPHIA, PA, US; 1 January 2004 (2004-01-01), BRANEN JILL K ET AL: "Enhancement of nisin, lysozyme, and monolaurin antimicrobial activities by ethylenediaminetetraacetic acid and lactoferrin." XP002316393 Database accession no. PREV200400138837 & INTERNATIONAL JOURNAL OF FOOD MICROBIOLOGY, vol. 90, no. 1, 1 January 2004 (2004-01-01), pages 63-74, ISSN: 0168-1605

## Description

### FIELD OF THE INVENTION

The present invention is generally related to a composition and method to reduce the microbial contamination on organic matter, such as processed meat, fruits and vegetables, plant parts; and other inanimate surfaces such as textiles and stainless steel. Such compositions can also be used in dental applications to reduce the microbial concentration in the mouth, for example.

### BACKGROUND

Food borne diseases cause significant illness and death each year, with direct and indirect medical costs estimated by some sources to be over 1 billion a year. Common food pathogens include *Salmonella, Listeria monocytogenes, Escherichia coli* 0157:H7, *Campylobacter jejuni, Bacillus cereus,* and Norwalk-like viruses. Outbreaks of food borne diseases typically have been associated with contaminated meat products, raw milk, or poultry products but fruits and vegetables can also serve as sources of food borne illness. Surfaces, containers, and other substrates can be a source of contamination in food. Recalls of food products, such as ground beef, hot dogs, alfalfa sprouts, and orange juice, show a need for a broad spectrum antimicrobial solution that is food grade, and cost effective.

Compositions used to reduce the microbial contamination in and on food as well as other surfaces have typically involved use of materials such as organic acids and chlorine compounds, such as sodium hypochlorite, that at higher concentrations may affect the properties of the surface treated. Compositions using fatty acid monoesters have been used in recent years to reduce microbial load on food such as poultry, as described in U.S. Patent Nos. 5,460,833 and 5,490,992, and fruit and vegetables, as described in International Publication No. WO 200143549A. Fatty acid monoesters have also been used in dried compositions on textiles, as described in Applicants' Assignee's Co-pending U.S. Patent Application Serial No. 09/572,549, filed on May 17, 2000. They have also been used on contact lenses, as described in U.S. Patent No. 4,485,029. The fatty acid monoesters in these compositions have limited stability in the presence of other components.

U.S. Patent No. 5,804,549 discloses compositions consisting essentially of the lanthionine containing bacteriocin, nisin in combination with glycerol monolaurate, and the use of such composition for the treatment of bacterial infections of the genus Helicobacter. These formulations are directed to treatment in the gastrointestinal tract by affecting the mucous System that protects the microbes. EP0629347 discloses antimicrobial mixtures comprising lactoferrin (hydrolysate) or lactoferrin-derived antimicrobial peptides and glycerin fatty esters.

### SUMMARY

The invention provides an antimicrobial composition as defined in claims 1 or 2, kits containing the composition as defined in claim 9, methods of using the composition as defined in claim 12 and methods of applying the compositions to a substrate as defined in claim 19.

In one aspect, the present invention provides an antimicrobial composition that includes: an antimicrobial lipid component (preferably, a major amoimt of an antimicrobial lipid component) that includes a compound selected from the group consisting of a (C7-C14) saturated fatty ether of a polyhydric alcohol, a (C8-C22) unsaturated fatty ether of a polyhydric alcohol, an alkoxylated derivative thereof, and combinations thereof, wherein the alkoxylated derivative has less than 5 moles of alkoxide per mole of polyhydric alcohol (preferably, the antimicrobial lipid component does not include a glycerol monoester); an enhancer component that includes a compound selected from the group consisting of iron-binding proteins and derivatives thereof, siderophores, and combinations thereof; and optionally a surfactant.

In another aspect, the compositions may optionally also contain a surfactant (i.e., one or more surfactants). The surfactants can be chosen based on the anticipated use of the composition. Suitable surfactants include acyl lactylate salts, dioctyl sulfosuccinate salts, lauryl sulfate salts, dodecylbenzene sulfonate salts, and salts of (C8-C18)fattyacids.

In a further aspect of the present invention, the compositions containing food-grade components preferably exhibit effective antimicrobial activity without detrimentally affecting the taste, texture, color, odor, or appearance of food and food products. This may be evaluated by using a blind taste test. For food that is normally cooked, such as hamburger, blind taste testing should be conducted on the cooked food. The treated food is considered to have no effect on taste, texture, color, odor, or appearance of food and food products, if there is no Statistical difference between the treated product and a control untreated product.

In another aspect, compositions containing components that are generally recognized as food grade (GRAS), such as many of the esters and enhancers of the present invention, preferably do not pose significant harmful toxicology or environmental problems. Many of the compositions of the present invention can also be readily handled at a processing plant and are compatible with processing equipment.

In another aspect, the present invention also includes a process of disinfecting foods or other surfaces. The method includes contacting the food or surface with a composition of the present invention. For certain embodiments, the composition is concentrated and the method includes diluting the composition before application to a substrate. In certain embodiments, a method is provided that includes applying an antimicrobial lipid component, and applying an enhancer component, in one or more parts. When two or more parts are applied, for example, the enhancer component can be applied before or after the antimicrobial lipid component.

In one embodiment, the present invention provides a method of applying an antimicrobial composition to a substrate. The method includes applying to the substrate a major amount of an antimicrobial lipid component including a compound selected from the group consisting of a fatty ether of a polyhydric alcohol, alkoxylated derivatives thereof, and combinations thereof; and applying to the substrate an enhancer component including a compound selected from the group consisting of iron-binding proteins and derivatives thereof, siderophores, and combinations thereof. The enhancer component can be applied simultaneously with, before, or after the antimicrobial lipid component.

In another aspect of the invention, preferably at least a one-log average reduction of total aerobic bacteria count (i.e., many of which can cause food to spoil) can be achieved on substrates (e.g., food products) using the formulations and methods disclosed herein. This can be determined according to the method described in Examples 5-7 using a sample of ground beef having an initial native bacteria concentration of 10000 -100,000 bacteria/gram ground beef when sufficient composition is applied such that 1% antimicrobial lipid is applied to ground beef. More preferably the compositions of this invention achieve at least 2 log average reduction, and even more preferably at least 3 log average reduction. Most preferably, compositions of the present invention achieve complete eradication of the native bacteria (such that the bacterial level is non-detectable).

In particular formulations, the compositions are not inactivated by organic matter. That is, compositions of the present invention are active in the presence of blood, serum, fats, and other organic matter typically found on food, and known to inactivate other antimicrobials such as iodine and quats.

In yet another aspect, the invention features a kit that includes a first container a first container that includes the antimicrobial lipid component and a second container that includes the enhancer component

One or both containers in the kit may also optionally contain a surfactant. The kit further can include a label or package insert indicating that contents of the first container and the second container are preferably mixed to produce an antimicrobial formulation that is effective for reducing microbial contamination. The label or package insert further can indicate that the antimicrobial formulation can be diluted before application to food, food products, or inanimate surfaces.

### Definitions

"Major amount" means a component present in a concentration higher than any other individual component.

"Enhancer" means a component that enhances the effectiveness of the antimicrobial lipid such that when either the composition without the antimicrobial lipid or the composition without the enhancer component are used separately, they do not provide the same level of antimicrobial activity as the composition as a whole. For example, an enhancer in the absence of the antimicrobial lipid may not provide any appreciable antimicrobial activity. The enhancing effect can be with respect to the level of kill, the speed of kill, and/or the spectrum of microorganisms killed, and may not be seen for all microorganisms. In fact, an enhanced level of kill is most often seen in Gram negative bacteria such as Escherichia coli. An enhancer may be a synergist that when combined with the remainder of the composition causes the composition as a whole to display an activity greater than the sum of the activity of the composition without the enhancer component and the composition without the antimicrobial lipid.

"Microorganism" or "microbe" refers to bacteria, yeast, mold, fungi, mycoplasma, as well as viruses.

"Shelf-Life" means a period of time it takes for a processed food to spoil. For example, beef can be considered to be spoiled if the bacterial count for an area of skin (one square centimeter) is equal to or greater than 10⁷ (colony forming units per square centimeter).

"Vehicle" means a carrier for the components of a composition. In antimicrobial compositions, the vehicle is typically the component present in a major amount.

Antimicrobial "activity" includes activity against microbes, including but not limited to, gram-negative bacteria and gram-positive bacteria, fungi, fungal spores, yeast, mycoplasma organisms, and lipid-coated viruses.

The terms "comprises" and variations thereof do not have a limiting meaning where these terms appear in the description and claims.

As used herein, "a," "an," "the," "at least one," and "one or more" are used interchangeably.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list. Other features and advantages of the invention will be apparent from the following detailed description, and from the claims.

### DETAILED DESCRIPTION OF ILLUSTRATIVE EMBODIMENTS

The present invention comprises antimicrobial compositions (some of which are in concentrated form), and methods of use of these compositions.

In one embodiment, a concentrated antimicrobial composition includes an antimicrobial lipid component including one or more compounds selected from the group consisting a fatty ether of a polyhydric alcohol, alkoxylated derivatives thereof, and combinations thereof. The compositions further include an enhancer component including one or more compounds selected from the group consisting of, iron-binding proteins and derivatives thereof, siderophores, and combinations thereof.

The compositions of the present invention may further include other additives, including surfactants and flavorants and flavor masking agents. For those compositions that include a major amount of the antimicrobial lipid that is liquid at room temperature, the antimicrobial lipid serves as both the active antimicrobial agent and a vehicle for the other components of the antimicrobial composition.

The formulations can be used to treat a wide variety of substrates that are or may be contaminated by microorganisms. For example, the compositions can be used to treat steel, glass, aluminum, wood, paper, polymeric materials, formica, and other counter top surfaces, tile, ceramics, rubber, paper, and textiles such as cotton, nylon, polypropylene non-wovens, and linen. Other uses for the compositions, such as food and medical applications, are described in Applicants' Assignee's Co-pending Patent Application Serial Nos. 10/659,584 and 10/659,571, filed on September 9, 2003. Still other uses for the compositions include dental applications.

The antimicrobial lipid component includes one or more compounds selected from the group consisting of a fatty ether of a polyhydric alcohol, alkoxylated derivatives thereof, and combinations thereof. In certain embodiments, the antimicrobial lipid component includes a compound selected from the group consisting of a (C7-C 14)saturated fatty ether of a polyhydric alcohol (preferably, a (C8-C14)saturated fatty ether of a polyhydric alcohol), a (C8-C22)unsaturated fatty ether of a polyhydric alcohol (preferably, a (C12-C22)unsaturated fatty ether of a polyhydric alcohol), an alkoxylated derivative thereof, and combinations thereof, wherein the alkoxylated derivative has less than 5 moles of alkoxide per mole of polyhydric alcohol.

Certain embodiments include a saturated fatty ether of a polyhydric alcohol, an unsaturated fatty ether of a polyhydric alcohol, an alkoxylated derivative thereof, and combinations thereof, wherein the alkoxylated derivative has less than 5 moles of alkoxide per mole of polyhydric alcohol.

In particular, formulations of the invention can reduce the number of food-borne human pathogens in meat. For example, they can be used as sprays and dips to treat meat carcasses such as beef, pork, poultry, fish, and lamb carcasses. They can also be used as sprays and dips to treat further processed meat such as ground beef, ground pork, ground chicken, ground turkey, hot dogs, sausages and lunch meats. Human food-borne pathogens killed by the formulations disclosed include, for example, E. coli 0157:H7, *Listeria monocytogenes,* and *Salmonella serovars*.

Not only can the formulations of the present invention be used to remove human pathogens from meat and meat products, they can also be used to help protect other foods, such as plants and plant parts, from human pathogens and other pathogens that produce spoilage and adversely effect the quality and shelf life of fruits and vegetables.

Generally, the components in the composition, as a whole, provide antimicrobial (including antiviral, antibacterial, or antifungal) activity having a spectrum of sufficient breadth to kill, or reduce the number to an acceptable level, of essentially most pathogenic or undesired bacteria, fungi, yeasts and lipid coated viruses. It should be understood that in the compositions of the present invention, the concentrations or amounts of the components, when considered separately, may not kill to an acceptable level, or may not kill as broad a spectrum of undesired microorganisms, or may not kill as fast; however, when used together such components provide an enhanced (preferably synergistic) antimicrobial activity (as compared to the same components used alone under the same conditions).

Those of ordinary skill in the art will readily determine when a composition of the present invention provides enhanced or synergistic antimicrobial activity using assay and bacterial Screening methods well known in the art. One readily performed assay involves exposing selected known or readily available viable bacterial strains, such as *Escherichia coli, Staphylococcus spp., Streptococcus spp., Pseudomonas spp.,* or *Salmonella spp.,* to a test composition at a predetermined bacterial burden level in a culture media at an appropriate temperature. After a sufficient contact time, an aliquot of a sample containing the exposed bacteiia is collected, diluted, neutralized, and plated out on a culture medium such as agar. The plated sample of bacteria is incubated for about forty-eight hours and the number of viable bacterial colonies growing on the plate is counted. Once colonies have been counted, the reduction in the number of bacteria caused by the test composition is readily determined. Bacterial reduction is generally reported as log₁₀ reduction determined by the difference between the log₁₀ of the initial inoculum count and the log₁₀ of the inoculum count after exposure.

Preferably, compositions of the invention demonstrate at least a one-log average reduction of total aerobic bacteria count when used on a substrate. To differentiate between enhanced activity and synergistic activity a checkerboard assay can be performed.

Preferred compositions of the present invention may be physically stable. As defined herein "physically stable" compositions are those that do not significantly change due to substantial precipitation, crystallization, phase separation, and the like, from their original condition during storage at 23 °C for at least 3 months, and preferably for at least 6 months. Particularly preferred compositions are physically stable if a 10-milliliter (10-ml) sample of the composition when placed in a 15-ml conical-shaped graduated plastic centrifuge tube (Corning) and centrifuged at 3,000 revolutions per minute (rpm) for 10 minutes using a Labofuge B, model 2650 manufactured by Heraeus Sepatech GmbH, Osterode, West Germany (2275 x g) has no visible phase separation in the bottom or top of the tube.

Preferred compositions of the present invention may exhibit good chemical stability.

Preferred compositions retain at least 85%, more preferably at least 90%, even more preferably at least 92%, and even more preferably at least 95%, of the antimicrobial lipid component after aging for 4 weeks at 50°C (an average of three samples). The most preferred compositions retain an average of at least 97% of the antimicrobial lipid after aging for 4 weeks at 50°C in a sealed container.

The percent retention is understood to mean the amount of antimicrobial lipid component retained comparing the amount remaining in a sample aged in a sealed container that does not cause degradation to an identically prepared sample (preferably from the same batch) to the actual measured level in a sample prepared and allowed to sit at room temperature for one to five days. For compositions that are meant to be in multiple parts, the part comprising the antimicrobial fatty acid ester preferably exhibits the above stability.

### Antimicrobial Formulations

Antimicrobial formulations of the invention may include one or more fatty ethers, or alkoxylated derivatives thereof, one or more enhancers, and optionally one or more surfactants. The compositions can be used for reducing levels of microorganisms, including gram-negative and gram-positive bacteria, viruses, fungi and fungi spores on plants and plant parts, meat and other foods as well as on inanimate surfaces. As used herein, "reducing levels of microorganisms" includes inhibiting microbial growth, promoting microbial death, and removing microorganisms from the surfaces of plants or plant parts, meat and other foods as well as from inanimate surfaces.

Preferred formulations of the present invention have a pH of no higher than 6, and more preferably, they have a pH of 4.5-5.5, when the composition is mixed in water at the concentration suitable for application to food.

### Antimicrobial Lipids

The antimicrobial lipid is that component of the composition that provides at least part of the antimicrobial activity. That is, the antimicrobial lipid has at least some antimicrobial activity for at least one microorganism. It is generally considered the main active component of the compositions of the present invention. The antimicrobial lipid may include one or more fatty ethers of a polyhydric alcohol, or alkoxylated derivatives thereof (of either or both of the ester and ether), or combinations thereof. More specifically, the antimicrobial component can include one or more compounds selected from the group consisting of a (C7-C14) saturated fatty ether of a polyhydric alcohol (preferably, a (C8-20 C14)saturated fatty ether of a polyhydric alcohol), a (C8-C22)unsaturated fatty monoether ofa polyhydric alcohol (preferably, a (C12-C22)unsaturated fatty monoether ofa polyhydric alcohol), an alkoxylated derivative thereof, and combinations thereof.

A fatty ether of a polyhydric alcohol is preferably of the formula (R³-0)ₙ-R⁴, wherein R³ is a (C7-C12) saturated aliphatic group (preferably, a (C8-C12) saturated aliphatic group), or a (C8-C22) unsaturated (preferably, a (C12-C22) unsaturated, including polyunsaturated) aliphatic group, R⁴ is the residue of glycerin, sucrose, or propylene glycol, and n = 1 or 2. For glycerin and propylene glycol n = 1, and for sucrose n = 1 or 2. Preferred fatty ethers are monoethers of (C7-C12)alkyl groups (preferably, (C8-C12)alkyl groups).

Exemplary fatty monoethers would include, but are not limited to laurylglyceryl ether, caprylglycerylether, caprylylglyceryl ether, laurylpropylene glycol ether, caprylpropyleneglycol ether, and caprylylpropyleneglycol ether. Other fatty monoethers include glycerin and propylene glycol monoethers of oleyl (18:1) linoleyl (18:2), linolenyl (18:3), and arachonyl (20:4) unsaturated and polyunsaturated fatty alcohols. Fatty monoethers that are suitable for use in the present composition include laurylglyceryl ether, caprylglycerylether, caprylyl glyceryl ether, laurylpropylene glycol ether, caprylpropyleneglycol ether, caprylylpropyleneglycol ether, and combinations thereof.

The alkoxylated derivatives of the aforementioned fatty ethers (e.g., one which is ethoxylated and/or propoxylated on the remaining alcohol group(s)) also have antimicrobial activity as long as the total alkoxylate is kept relatively low. Preferred alkoxylation levels are disclosed in U.S. Pat. No. 5,208,257 (Kabara). In the case where the ethers are ethoxylated, the total moles of ethylene oxide is preferably less than 5, and more preferably less than 3.

The fatty ethers of polyhydric alcohols can be alkoxylated, preferably ethoxylated and/or propoxylated, by conventional techniques. Alkoxylating compounds are preferably selected from the group consisting of ethylene oxide, propylene oxide, and mixtures thereof, and similar oxirane compounds.

The compositions of the present invention may include one or more fatty ethers, or alkoxylated fatty ethers at a suitable level to produce the desired result. When diluted with a vehicle, the antimicrobial compositions can include a total amount of such material of at least 0.01 percent by weight (wt-%), preferably at least 0.10%, and more preferably at least 1 wt-%, based on the total weight of the composition.

Preferred compositions of the present invention that include one or more fatty monoethers, or alkoxylated derivatives thereof can also include a small amount of a di- or tri-fatty acid ester (i.e., a fatty acid di- or tri-ester), a di- or tri-fatty ether (i.e., a fatty di- or tri-ether), or alkoxylated derivative thereof. For monoethers, or alkoxylated derivatives of propylene glycol, preferably there is no more than 40% of the di- functional material. For monoethers, or alkoxylated derivatives of glycerin, preferably there is only a small amount of the di- or tri- functional material. In the case of fatty monoethers of glycerin, preferably there is no more than 15 wt-%, more preferably no more than 10 wt-%, even more preferably no more than 7 wt-%>, even more preferably no more than 6 wt-%, and even more preferably no more than 5 wt-% of a diether, triether, or alkoxylated derivatives thereof present, based on the total weight of the antimicrobial lipid present in the composition. As used herein, "fatty" refers to a straight or branched chain alkyl or alkylene moiety having 6 to 14 (odd of even mimber) carbon atoms, unless otherwise specified.

Antimicrobial lipids that are liquid at or above 4°C can serve as both the vehicle and the antimicrobial active. These concentrated compositions may both increase efficacy and at the same time give stable compositions and reduce costs of use.

In certain embodiments, the antimicrobial lipid component includes a fatty ether of a polyhydric alcohol, alkoxylated derivatives thereof, or combinations thereof.

In certain embodiments, the antimicrobial lipid component does not include a glycerol monoester.

In certain embodiments, the antimicrobial lipid component includes a compound selected from the group consisting of a saturated fatty ether of a polyhydric alcohol, an unsaturated fatty ether of a polyhydric alcohol, alkoxylated derivatives thereof, and combinations thereof, wherein the alkoxylated derivative has less than 5 moles of alkoxide per mole of polyhydric alcohol.

### Enhancers

Compositions of the present invention include an enhancer (preferably a synergist) to enhance the antimicrobial activity. The enhancers may be selected from the group consisting of iron-binding proteins and derivatives thereof, siderophores, and combinations thereof. The preferred enhancers are selected from the following classes of compounds discussed below.

Iron-binding compounds include both small siderophores and iron-binding proteins and derivatives thereof.

Suitable iron-binding siderophores include organic molecules having a molecular weight of less than 1000 daltons (typically, 400-1000 daltons) that are released by bacteria in iron-limiting situations to complex ferric iron and prevent precipitation of iron oxyhydroxides in the natural environment. They are composed of hydroxamate and phenolate derivatives, which provide high affmity complexation sites for ferric iron. Examples of such molecules are high affinity ferric ion chelators synthesized by bacteria, which may include, but are not limited to, enterochelin (enterobactin), vibriobactin, Anguibactin, pyochelin, Pyoverdin, Mycobactin, Exochelins, Aerobactin, and Desferrioxamine.

Suitable iron-binding proteins include lactoferrin and derivatives thereof, particularly peptides derived therefrom (e.g., lactoferricin B, lactoferricin H, derived by enzymatic cleavage of lactoferrin and activin) and transferrin. In certain embodiments, lactoferrin is a preferred enhancer.

Suitable sugars can include both monosaccharides and disaccharides. Suitable monosaccharides include, but are not limited to, mannose, xylose, maitose, sorbose, and their corresponding sugar alcohols mannitol, xylitol, maltitol, and sorbitol. In certain preferred embodiments, the sugar is selected from the group consisting of mannose, xylose, mannitol, xylitol, and combinations thereof. In certain embodiments, the sugar is a disaccharide of xylitol and glucose. For disaccharides, at least one of the sugars is preferably one of the suitable monosaccharides listed herein. The second sugar unit may be selected from any suitable sugar commonly used in food products, such as but not limited to, glucose, fructose, mannose, xylose, galacose, sorbose, and sorbitol.

It should be understood that various combinations of enhancers can be used if desired. In some embodiments, significant results can be obtained through the use of a combination of enhancers.

Certain compositions of the present invention include at least two enhancers, and preferably at least three enhancers, of different classes of compounds. For example, certain embodiments include an enhancer selected from the group consisting of iron binding proteins, siderophores, and combinations thereof with at least one other enhancer of at least one different class of compound.

Alternatively, certain embodiments include an enhancer selected from the group consisting of iron binding proteins, siderophores, and combinations thereof with at least two other enhancers of at least two different classes of compound. Such other enhancers can be selected from the group consisting of bacteriocins, antimicrobial enzymes, sugars, sugar alcohols, and combinations thereof. In one embodiment, the enhancer component includes nisin and lactoferrin. In one embodiment, the enhancer component comprises nisin, lactoferrin, and a sugar and/or a sugar alcohol.

Compositions that include nisin, particularly in combination with various non-bactericidal agents, have been shown to be highly active against various species of Gram-positive and Gram-negative bacteria (see, e.g., U.S. Pat. Nos. 5,135,910; 5,217,950, and 5,260,271). More recently, bactericidal activity of nisin, in the presence of chelators, has been described against additional Gram-negative bacteria, including Helicobacter pylori (see, e.g., U.S. Pat. Nos. 5,304,540 and 5,334,582). Glycerol monolaurate and nisin, which alone are suboptimal in their bactericidal effect, mutually enhance the bactericidal activity of one another in combination against strains of the genus Helicobacter. The formulations of nisin with glycerol monolaurate, however, can be ineffective as antimicrobial compositions applied to meat and other food products. In certain preferred embodiments, nisin is not included.

Other classes of enhancer compounds, such as an organic acid, a chelating agent, a phenolic compound, or an alcohol, may also be added to the antimicrobial compositions. Suitable organic acids can include, for example, lactic acid, tartaric acid, adipic acid, succinic acid, citric acid, ascorbic acid, glycolic acid, malic acid, mandelic acid, acetic acid, sorbic acid, benzoic acid, and salicylic acid. In certain embodiments, the enhancer component includes an organic acid and a compound selected from the group consisting of bacteriocins, antimicrobial enzymes, sugars, sugar alcohols, iron-binding proteins and derivatives thereof, siderophores, and combinations thereof. In one embodiment, the enhancer component includes an organic acid, lactoferrin, and either a sugar, a sugar alcohol, or both.

Suitable chelating agents can include, for example, sodium acid pyrophosphate, acidic sodium hexametaphosphate (such as SPORIX acidic sodium hexametaphosphate), ethylenediaminetetraacetic acid (EDTA) and salts thereof. Suitable alcohols can be, for example, ethanol, isopropanol, or long chain alcohols such as octanol or decyl alcohol. Phenolic compounds such as butylated hydroxyanisole, butylated hydroxytoluene, and tertiary butyl hydroquinone, for example, enhance the activity of the fatty acid monoesters as do benzoic acid derivatives such as methyl, ethyl, propyl, and butyl parabens. In certain preferred embodiments, the organic acid enhancer is benzoic acid and the phenolic compound enhancer is methyl paraben (4-hydroxybenzoic acid methyl ester).

### Surfactants

Compositions of the present invention may include a surfactant to emulsify the composition and to help wet the surface to aid in contacting the microorganisms. As used herein the term "surfactant" means an amphiphile which is defined as a molecule possessing both polar and nonpolar regions which are convalently bound. The term is meant to include soaps, detergents, emulsifiers, surface active agents and the like. The surfactant can be cationic, anionic, nonionic, or zwitterionic. This includes a wide variety of conventional surfactants; however, certain ethoxylated surfactants may reduce or eliminate the antimicrobial efficacy of the antimicrobial lipid. The exact mechanism of this is not known and not all ethoxylated surfactants display this negative effect. For example, poloxamer polyethylene oxide/polypropylene oxide surfactants have been shown to be compatible with the antimicrobial lipid component, but ethoxylated sorbitan fatty acid esters such as those sold under the trade name TWEEN by ICI have not been compatible in some formulations. It should be noted that these are broad generalizations and the activity can be formulation dependent, i.e., based on the selection and amount of both antimicrobial lipid and ethoxylated surfactant used. One skilled in the art can easily determine compatibility of a surfactant by making the formulation and testing for antimicrobial activity as described in the Examples Section. Combinations of various surfactants can be used if desired.

Anionic surfactants, cationic surfactants, nonionic surfactants and amphoteric surfactants may be used to make suitable emulsions of the antimicrobial fatty acid esters. For example, an antimicrobial formulation can include anionic surfactants such as acyl lactylate salts, dioctyl sulfosuccinate salts, lauryl sulfate salts, dodecylbenzene sulfonate salts, and salts of (C8-C18)fatty acids. Suitable salts include sodium, potassium, or ammonium salts. Acyl lactylates include, for example, calcium or sodium stearoyl-2-lactylate, sodium isostearoyl-2-lactylate, sodium lauroyl-2-lactylate, sodium caproyl lactylate, sodium cocoyl lactylate, and sodium behenoyl lactylate. Nonionic surfactants include glycerol esters such as decaglyceryl tetraoleate; sorbitan esters such as sorbitan monolaurate, commercially available under the trade designation SPAN 20 from Uniquema International, Chicago, IL; and block copolymers of polyalkylene oxide, e.g., polyethylene oxide and polypropylene oxide available under the trade designations PLURONIC and TETRONIC from BASF (Parsippany, NJ). Dioctyl sodium sulfosuccinate is commercially available under the trade designation GEMTEX SC40 surfactant (40% dioctyl sodium sulfosuccinate in isopropanol) from Finetex Inc., Spencer, North Carolina. Sodium caproyl lactylate is commercially available under the trade designation PATIONIC 122A from RITA (Woodstock, IL). Sodium lauryl sulfate is commercially available from Stepan Chemical Co., Northfield, IL.

Other surfactants that may be suitable for use in the antimicrobial compositions of the present invention are listed in Applicants' Assignee's Co-pending Patent Application Serial No. 10/659,584, filed on September 9, 2003.

### Applications with Food

The formulations of the invention are particularly useful for reducing levels of food borne human pathogens, including *Escherichia coli* 0157:H7, *Salmonella* serotypes, including *S.* typhimurium, *Listeria* (e.g., *L. monocytogenes), Campylobacter* (e.g., *C.jejuni), Shigella* species, and *Bacillus cereus.*

Monoglycerides useful in the invention typically are available in the form of mixtures of unreacted glycerol, monoglycerides, diglycerides, and triglycerides. Thus, it is preferred to use materials that contain a high concentration, e.g., greater than 60 wt-% of monoglyceride. In some compositions, the desired materials will contain concentrations greater than 85 wt-% or 90 wt-% of monoglyceride. Examples of particularly useful commercially available materials (not forming part of the invention) include glycerol monolaurate (GML), available from Med-Chem Laboratories, East Lansing, MI, under the tradename LAURICIDIN, glycerol monocaprylate (GM-C8) and glycerol monocaprate (GM-C10) available from Riken Vitamin Ltd., Tokyo, Japan under the tradenames POEM M-100 and POEM M-200, respectively, and those available from the Henkel Corp. of Germany under the tradename "MONOMULS 90 L-12". Propylene glycol monocaprylate (PG-C8), propylene glycol monocaprate (PG-C10), and propylene glycol monolaurate (PG-C12) are available from Uniquema International, Chicago, IL.

In food applications, the enhancers are food grade, GRAS listed, and/or FDA-cleared food additives. The amounts of enhancer in the present invention may be up to 20.0 wt-%, and preferably 1.0 wt-% to 10.0 wt-%. In other embodiments, such as those that include a vehicle, the enhancer may include 0.01 wt-% to 1.0 wt-%, and preferably 0.01 wt-% to 0.5 wt-%. Lower concentrations of enhancer may be necessary, in part, in order to avoid undesired changes or alterations to the taste, texture, color, odor or appearance of the food. Depending on the particular enhancer used, it can either be formulated directly into the concentrate vehicle if soluble and stable in the esters or it can be packaged separately in a suitable solvent.

In most compositions, food grade and/or GRAS surfactants may be used in amounts which provide a concentrated composition of 1.0 wt-% to 30.0 wt-%, and preferably 4.0 wt-% to 12. 0 wt-%. In other embodiments that include a vehicle, the composition may provide a surfactant concentration of 0.001 wt-% to 1.0 wt-%, and preferably 0.01 wt-% to 0.5 wt-%.

The concentration of the aforementioned components required for effectively inhibiting microbial growth depends on the type of microorganism targeted and the formulation used (e.g., the type of antimicrobial lipid, enhancer, and surfactants that are present). The concentrations or amounts of each of the components, when considered separately, may not kill as great a spectrum of pathogenic or undesired microorganisms, kill them as rapidly, or reduce the number of such microorganisms to an acceptable level, as the composition as a whole. Thus, the components of the formulation, when used together, provide an enhanced or synergistic antimicrobial activity to the meat, plants or plant parts, or other treated surfaces when compared to the same components used alone and under the same conditions. Acceptable levels of antimicrobial activity typically exceed 1-log reduction in or on a food, or other surface.

Effective amounts of each component can be readily ascertained by one of skill in the art using the teachings herein and assays known in the art. The compositions of the invention may be prepared and used directly or can be diluted to prepare a nonaqueous or aqueous solution, emulsion or suspension before use. Suitable vehicles for preparing the solutions or suspensions are typically acceptable to regulatory agencies such as the FDA and the U.S. Environmental Protection Agency (EPA). Particularly acceptable vehicles include water, propylene glycol, polyethylene glycol, glycerin, ethanol, isopropanol, and combinations thereof. Alternatively, one or more antimicrobial lipids may function as the vehicle.

In preferred embodiments, the fatty acid monoglyceride (not forming part of the invention) is 0.001 wt-% to 30 wt-%, the enhancer is 0.001 wt-% to 30 wt-%, and one or more surfactants are 0.001 wt-% to 30 wt-% of the antimicrobial formulation. For example, a ready-to-use formulation can include 0.01 wt-% to 5.0 wt-% of a fatty acid monoester, (not forming part of the invention) 0.5 wt-% to 30 wt-% of an enhancer, and 0.5 wt-% to 5.0 wt-% of a surfactant. In particular, a ready-to-use formulation can include 0.2 wt-% to 2.0 wt-% of the fatty acid monoester, (not forming part of the invention) 0.1 wt-% to 25.0 wt-% of the enhancer, and 0.1 wt-% to 1.5 wt-% of one or more surfactants.

Additional components of the antimicrobial formulations can include, for example, food-grade coating agents such as beeswax, paraffin, carnauba, candelilla and polyethylene waxes; other coating materials including resins, shellac, wood rosin, com zein; and components that protect the formulations from UV inactivation or degradation, colorants, odor-enhancing agents, viscosity control agents such as gum tragacanth, gum accacia, carageenans, Carbopols (B.F. Goodrich, Cleveland, Ohio), guar gum, and cellulose gums; anti-foaming agents such as silicone anti-foams, e.g., polydimethylsiloxanes (Dow Corning, Midland, MI), sticking agents, or flavorants such as natural oils or artificial sweeteners.

Antimicrobial formulations used in food applications typically exhibit increased antimicrobial efficacy with increased temperatures at application.

### Treating Meat and Meat Products

The composition of the present invention may be prepared by combining the above described components using processes and procedures well known to those of ordinary skill in the art.

In some embodiments, the antimicrobial lipid can be applied in a separate step from applying the enhancer. The compositions of the present invention may be used in a food processing plant in a variety of suitable ways during various stages of the process. For example, the present composition may be applied to meat products, such as beef carcasses, beef trim, beef primals, or ground meat as a spray, a rinse, or a wash solution. The meat products may also be dipped in the composition. In addition, the present invention has a wide useful temperature range which allows the composition to be used at different stages in a process plant. For example, the composition may be used at elevated temperatures to disinfect beef carcasses and at cold (4-5°C) temperatures to disinfect ground beef and beef trim. The compositions of the present invention may also be useful in the products and processes disclosed in U.S. Patent Nos. 5,460,833 and 5,490,992.

### Treating Plauts and Plant Parts

Using the formulations of the present invention, levels of plant pathogens may be reduced on the surfaces of plants and plant parts, which can extend shelf life of the plants and plant parts. Non-limiting examples of plant pathogens include *Erwinia carotovora, Fusarium* species, *Botrytis* species, *Phytopthera* species, *Phoma* species, *Verticilium* species, *Penicillium* species, and *Colletotrichum* species. The formulations of the invention may also be effective at reducing viability of spores on surfaces of plants and plant parts, such as spores from penicillium fungi.

Formulations of the invention can be applied to plants and plant parts by, for example, spraying, dipping, wiping, brushing, sponging, or padding. The formulation can be applied to a portion of or over the entire exterior surface of a plant or plant part. In most applications, the entire surface of the plant or plant part is fully wetted with the formulation. In some embodiments, the antimicrobial lipid can be applied in a separate step from applying the enhancer.

Formulations can be applied at temperatures ranging from 2°C to 90°C and are in contact with the surface of the plant or plant part for a time sufficient to reduce microbial levels (e.g., 10 seconds to 60 minutes). Typically, application time is reduced as temperature is increased. Heating the formulation to between 40°C and 65°C (e.g., 44-60°C, 46-58°C, 48-56°C, or 50-54°C) and applying to the surface while still warm may be particularly effective for reducing microbial levels on plants or plant parts. Also, if the plant or plant part is cooked, compositions of the present invention can be particularly effective. If present, the liquid vehicle can be removed from the surface of plant or plant part by, for example, air drying.

Suitable plants and plant parts may include raw agricultural commodities (i.e., non-processed products) and processed products. Non-limiting examples of raw agricultural commodities include alfalfa seeds, sprouts, cucumbers, melons, onions, lettuce, cabbage, carrots, potatoes, eggplants, citrus fruits such as grapefruits, lemons, limes, and oranges, bananas, pineapples, kiwis, and apples. Processed products include torn, sliced, chopped, shredded, or minced fruits or vegetables, as well as juice obtained from fruits or vegetables.

For example, a fruit such as an orange can be treated with an antimicrobial formulation of the invention, air-dried, then coated with a food-grade wax. This produces an orange having the antimicrobial formulation interposed between the orange and the food-grade coating. Alternatively, the antimicrobial formulation and a food-grade coating can be intermixed prior to application. In another alternative, the food-grade wax may be applied to fruit, such as an orange, and then the fruit can be treated with the antimicrobial composition over the wax. These may be conveniently applied as an aqueous dispersion. Suitable waxes are beeswax, cetyl palmitate, and the like.

The compositions of the present invention may also be useful in the products and processes disclosed in International Publication No. WO 200143 549A.

They may also be useful in treating food processing equipment, medical devices, cloth, paper, or any surface where a bactericidal activity is desired.

### Dental Applications

The present invention provides antimicrobial dental compositions and methods of using and making the compositions. Such compositions are useful for the treatment of oral diseases such as caries or periodontal disease caused by effective reduction, prevention or elimination of the causative bacterial species. Typically the compositions are applied topically to the oral hard or soft tissues or are compositions that are used to restore the oral hard tissues. Oral hard tissues include dental structure surfaces that include tooth structures (e.g., enamel, dentin, and cementum) and bone. Oral soft tissues include mucosal tissues, i.e., mucous membranes. Such compositions can provide effective reduction, prevention, or elimination of microbes, particularly bacteria, fungi, and viruses. In certain embodiments, the dental compositions of the present invention have a broad spectrum of activity.

Certain dental compositions of the present invention provide effective topical antimicrobial activity and are accordingly useful in the local treatment and/or prevention of conditions that are caused or aggravated by microorganisms (including viruses, bacteria, fungi, mycoplasma, and protozoa) on oral tissue or dental materials. Significantly, certain embodiments of the present invention have a very low potential for generating microbial resistance. Thus, such compositions can be applied multiple times over one or more days to treat oral surface infections or to eradicate unwanted bacteria. Furthermore, compositions of the present invention can be used for multiple treatment regimens on the same patient without the fear of generating antimicrobial resistance.

Dental compositions suitable for infection control of devices used in the mouth and then handled extraorally are also within the scope of this invention.

Dental compositions of the present invention include one or more antimicrobial lipid component such as a fatty ether of polyhydric alcohol, alkoxylated derivatives thereof etc.

Certain preferred dental compositions contain an enhancer or synergist selected from the group consisting of organic acids (for example, benzoic acid), sugars (such as xylose and mannose), sugar alcohols (such as xylitol), bacteriocins (such as nisin), proteins (such as lactoferrin), and combinations thereof. Other components that can be included are surfactants (such as diocyl sodium sulfosuccinate), hydrophilic components (such as a glycol, a lower alcohol ether, a short chain ester, and combinations thereof), and hydrophobic components. The compositions may be used in concentrated form or further combined in either an aqueous or nonaqueous vehicle before use.

A further aspect of this invention is a dental composition for chemo-mechanical or enzymatic removal of a carious lesion, which effectively eliminates any residual caries bacteria in the affected zone. In some embodiments a separate antimicrobial composition can be applied after removal of the carious lesion by the chemo-mechanical, enzymatic or purely mechanical means.

The antimicrobial compositions of the present invention may be used in the form of a spray, wash, rinse, liquid, paste, or powder to reduce the concentration of deleterious bacteria, e.g., Streptococcus mutans, in the mouth. Such dental products include, but are not limited to, oral rinses (e.g., mouthwashes), oral irrigational solutions, remineralization solutions, and the like. In another aspect, the compositions can be used for oral Prophylaxis, e.g., prophy pastes, prophy powder, sub-gingival cleansing, and the like. Compositions for use directly on a hard dental stracture include dentifrices (e.g., toothpastes), denture adhesives, and etchants.

Compositions of the present invention may also be used to provide antimicrobial protection of dental articles as well as dental equipment, such as, for example, dental impression trays, dental instruments, dental floss, dental picks, dental tape, dental packing (e.g., fibers), and the like.

### Articles of Manufacture

Formulations of the invention can be packaged into kits. Some antimicrobial lipids can be inherently reactive, especially in the presence of enhancers. Depending on the components chosen, the antimicrobial activity of the liquid composition may be reduced and shelf life may be shortened to less man one year.

Thus, the formulations may be packaged conveniently in a two-part system (kit) to increase stability. In one example of a two-part system, all components of the formulation, except the enhancer, are present in one container, while the enhancer is present in a separate container. Contents from each container are mixed together and may be diluted before treating the applicable food or surface.

In some embodiments, the antimicrobial formulation is packaged in a single container having separate compartments for storing various components-, e.g., the enhancer is in one compartment and the antimicrobial lipid, and optionally one or more surfactants, and a second enhancer are in a second compartment of the same container. Such two-compartment containers typically employ a breakable or displaceable partition between the two compartments. The partition then can be either broken or displaced to allow mixing. Alternatively, the container is configured such that a portion of the contents from each compartment can be removed, without mixing the entire contents of each compartment. See, for example, U.S. Patent Nos. 5,862,949, 6,045,254 and 6,089,389 for descriptions of two-compartment containers.

Unless otherwise defined, all technical and scientific terms used herein have the same meanings as commonly understood by one of ordinary skill in the art to which this invention belongs.

In case of conflict, the present specification, including definitions, will control. In addition, the examples are illustrative only and not intended to be limiting. The invention will be further described in the following examples, which do not limit the scope of the invention described in the claims.

### EXAMPLES

The following examples are intended to provide further details and embodiments related to the practice of the present invention. The following examples are offered for illustrative purposes to aid in understanding of the present invention and are not to be construed as limiting the scope thereof. All materials are commercially available unless otherwise stated or apparent. All parts, percentages, ratios, etc., in the examples are by weight unless otherwise indicated.

### GLOSSARY

| **Material** | **Nomenclature in the text** | **■ Supplier** |
|---|---|---|
| Glycerol monolaurate | GMLC12 | Med-Chem. Labs, MI |
| Propylene Glycol MonoCaprylate | PGMC8 | Uniqema, NJ |
| Sodium caproyl lactylate | Pationic 122A | RITA Chicago, IL |
| Sodium Lauroyl lactylate | Pationic 138C | RITA Chicago, IL |
| Sorbitan Monolaurate | Span 20 | Uniqema, NJ |
| 50% Dioctyl Sodium Sulfosuccinate inPEG-400 | DOSS | Cytec Industries/ West Paterson, NJ |
| Butylated Hydroxyanisole | BHA | EASTMAN, TN |
| PLURONIC P65 surfactant | PLURONIC P65 | BASF, NJ |
| Benzoic Acid | Benzoic Acid | Mallinckrodt, St. Louis, MO |
| Lactic Acid | Lactic Acid | PURAC, Lincolnshire, IL |
| Xylitol | Xylitol | Sigma-Aldrich/ St. Louis, MO |
| Xylose | Xylose | Avocado |
| D-Mannose | D-Mannose | Sigma-Aldrich/ St. Louis, MO |
| Mannitol | Mannitol | Sigma-Aldrich/ St. Louis, MO |
| Lactoferrin | Lactoferrin | DMV international, NY |
| Transferrin | Transferrin | Sigma-Aldrich/ St. Louis, MO |
| Nisin (40,000 unit/mg) | Nisin | Sigma-Aldrich/ St. Louis, MO |
| Colistin (Polymyxin E), Sulfate Salt, at least 15,000 units/mg | Colistin | Sigma-Aldrich/ St. Louis, MO |

### Examples 1-4 (comparative examples, not forming part of the invention) and Comparative Examples C1-C8: Antimicrobial efficacy on hard surfaces

Concentrate 1 was prepared by mixing 94 parts by weight of PGMC8 and 6 parts by weight DOSS. Example solutions were prepared by diluting Concentrate 1 to 0.5-1 wt-% in water with Mannitol or Lactoferrin or Nisin or Colistin, respectively, to prepare the example solutions. The proportions mixed are presented in Table 1 and Table 2 for the various enhancers. The solutions were shaken until a milky emulsion formed. The emulsion solutions were used immediately after being made. The pH values of all final solutions were in the range of 4.5-5. Comparative examples were prepared in the same manner with either the enhancer or the antimicrobial lipid not present.

**Table 1. Formulations for Examplesl-3 and Comparative Examples C1-C6. w/w%**

| Table 1 | Comp. Ex. C1 | Comp. Ex. C2 | Comp. Ex. C3 | Comp. Ex. C4 | Comp. Ex. C5 | Comp. Ex.C6 | Ex. 1 | Ex. 2 | Ex. 3 |
|---|---|---|---|---|---|---|---|---|---|
| Formulation | 2 | 3 | 4 | 5 | 6 | 7 | 8 | 9 | 10 |
| Concentrate 1 | | | | 1.0 | 0.9 | | 1.0 | 0.9 | 0.9 |
| Mannitol | | | | | | 15.0 | | | 15.0 |
| Nisin | | 0.15 | 0.10 | | | | | 0.15 | |
| Lactoferrin | 1.0 | | 1.0 | | | | 1.0 | | |
| Water | 99.0 | 99.5 | 98.9 | 99.0 | 99.1 | 85.0 | 98.0 | 98.9 | 84.1 |
| pH | 4.5-5 | | | | | | | | |

**Table 2. Formulations for Example 4 and Comparative Examples C7 and C8**

| Table 2 | Example 4 | Comparative Example C7 | Comparative Example C8 |
|---|---|---|---|
| | Formulation 13 | Formulation 11 | Formulation 12 |
| Concentrate 1 | 0.90 | 0.90 | |
| Colistin | 0.050 | | 0.050 |
| Water | 99.05 | 99.1 | 99.95 |
| pH | 4.5-5 | | |

### Inoculums and testing procedure:

The procedure from AOAC Official Method (AOAC Official Method 991.49, 6.2.05), Testing guidance Disinfectants Against *Pseudomonas aeruginosa*, was used for testing disinfectant against *Pseudomonas* (ATCC 9027) and AOAC Official Method 955.15: Testing Disinfectants against Staphylococcus aureus was used for testing disinfectants against the *Methicilum Resistant Staphylococcus Aureus* (MRSA) (ATCC# 33593). Initial Inoculums: *Pseudomonas:* 8.00 logs(lx 10⁸ colony-forming units (CFU) per milliliter (mL)); MRSA 8 log(lx 10⁸ CFU/mL).

In this test, hollow glass cylinders (penicylinders) were coated with the challenge bacteria. The bacteria were allowed to dry on the penicylinders for 1 hour (hr). The penicylinders with the dried bacteria were dipped into the example formulation for 24 hours, removed, and placed into neutralizer solution (D/E neutralizing broth) for 30 seconds and then put into Tryptic Soy Broth (TSB) containing glass tubes for 24 hours. At the end of 24 hours the tubes containing the penicylinders were evaluated for turbidity and scored as either growth or no growth. Ten hollow glass cylinders were tested per formulation. If 0 out of 10 tubes showed no growth, the formulation was rated "Pass." If more than 1 tube out of 10 tubes showed growth, the formulation was rated "Fall."

**Table 3. Antimicrobial formulations effect on Pseudomonas (ATCC 9027)**

| Table 3 | Comp. Ex. C1 | Comp. Ex. C2 | Comp. Ex.C3 | Comp. Ex. | Comp. Ex. C5 | Comp | Ex. 1 | Ex. 2 | Ex. 3 |
|---|---|---|---|---|---|---|---|---|---|
| Formulation | 2 | 3 | 4 | 5. | 6 | 7 | 8 | 9 | 10 |
| Fail tubes/ total tubes | 9/10 | 7/9 | 6/9 | 2/10 | 2/10 | 10/10 | 0/10 | 0/10 | 0/10 |
| Rating | Fail | Fail | Fail | Fail | Fail | Fail | Pass | Pass | Pass |

**Table 4. Antimicrobial formulation effect on MRSA (ATCC# 33593)**

| Table 4 | Comp. Ex. C7 | Comp. Ex. C8 | Example 4 |
|---|---|---|---|
| | Formulation 11 | Formulation 12 | Formulation 13 |
| Failed tubes /total tubes | 2/10 | 3/10 | 0/10 |
| Rating , | Fail | Fail | Pass |

### Examples 5-7(comparative examples, not forming part of the invention) and Comparative Examples C9-C10: Treatment of ground beef

Antimicrobial efficacy using Lactoferrin and Nisin as additional enhancers for ground beef treatment was evaluated.

### Determination of the native bacteria counts on ground beef

Ground beef purchased from the grocery store was tested immediately for native bacteria count by placing 11-gram (11-g) portions of the ground beef into separate Homogenizer Bag filtered #6469(3M, St. Paul, MN) with 99 milliliters (mL) of Letheen Broth (VWR Scientific, Batavia, IL) in each and stomached for 1 minute in a laboratory blender Stomacher 400 (Tekmar, Cincinnati, OH). Serial ten-fold sequential dilutions were made with Letheen broth. Samples were plated on PETRIFILM *Enterobacteriaceae* count plate (EB) (available from 3M, St. Paul, MN) and PETRIFILM Aerobic Count (AC) plate (available from 3M, St. Paul, MN). PETRIFILM plates were incubated for 24 +/- 2 hours at 35°C and counted as recommended on the package insert. Plates with counts that were within the counting range of the plates (25-250 CFU per PETRIFILM AC plate and 15-100 CFU per PETRIFILM EB plate) were used for analysis.

### Formulation prenaration and meat treatment

A concentrate of antimicrobial lipid (Concentrate 2) was made by adding the components listed in the Table 5 below into a glass container. The container was heated on a hot plate to 50-80 °C, and during heating the solution was constantly stirred (either by a magnet or a propeller stirring system). The solution was mixed until a homogenous, transparent, single-phase liquid resulted. This concentrate was used to treat the ground beef samples.

| Table 5 | Concentrate 2 |
|---|---|
| GML12 | 15.0 |
| PGMC8 | 45.0 |
| Pationic 122A | 10.0 |
| Span 20 | 15.0 |
| DOSS | 3.0 |
| BHA | 2.0 |
| Benzoic Acid | 10.0 |

The ground beef samples were treated with a two part system - Part A and Part B. Part A consisted of Concentrate 2 diluted in water to 40 wt-% and Part B consisted of an aqueous enhancer solution made by dissolving either 10 wt-% Lactoferrin or 1.6 wt-% Nisin in water or both 10 wt-% Lactoferrin and 1.6 wt-% Nisin in water.

Weighed amounts of ground beef were added into the KITCHEN-AID mixer equipped with a paddle mixing head. The Part B solution was placed in a pressure pot (23°C) connected to a spray nozzle. The solution was sprayed into the ground beef (5°C) contained in the mixer while mixing occurred with the paddle mixer. The sprayed meat contained about 2.5 wt-% of the aqueous solution. This delivered 0.25 wt-% Lactoferrin and/or 0.04 wt-% Nisin (800 IU) to the ground beef in 1.5 minutes (min). Samples were then sprayed with Part A (40 wt-% Concentrate 2). The sprayed meat contained about 2.5 wt-% aqueous solution (1 wt-% Concentrate 2 delivered into the meat samples). The total mixing time was 3 minutes. The treated ground beef samples were placed in the refrigerator again. Five sets of treatments were applied to the ground meat with the above-described spraying procedure. After the spray and mixing, the final weight percent in meat for each treatment was as listed in the Table 6 below.

**Table 6. Final component weight percentages in meat for Examples 5-7 and Comparative Examples 9 & 10**

| Table 6 | Comp. Ex. C9 | Ex. 5 | Ex. 6 | Ex. 7 | Comp. Ex. C10 |
|---|---|---|---|---|---|
| | Diluted Concentrate 2 Only | Diluted Concentrate 2 with Lactoferrin | Diluted Concentrate 2 with Nisin | Diluted Concentrate 2 with Lactoferrin and Nisin | Lactoferrin and Nisin Only |
| solution pH | 4.5 | 5.5 | 5.5 | 5.5 | 5.5 |
| GMLC12 | 0.150 | 0.150 | 0.150 | 0.150 | |
| PGMC8 | 0.450 | 0.450 | 0.450 | 0.450 | |
| Pationic 122A | 0.100 | 0.100 | 0.100 | 0.100 | |
| Span 20 | 0.150 | 0.150 | 0.150 | 0.150 | |
| DOSS | 0.030 | 0.030 | 0.030 | 0.030 | |
| Benzoic Acid | 0.100 | 0.100 | 0.100 | 0.100 | |
| BHA | 0.020 | 0.020 | 0.020 | 0.020 | |
| Lactoferrin | | 0.250 | | 0.250 | 0.250 |
| Nisin | | | 0.040 | 0.040 | 0.040 |

No color change was observed in any of these samples over the course of storage. After 24 hours, an 11-g aliquot of ground beef was weighed out and placed into Homogenizer Bag filtered with 99 mL of Letheen broth added to the samples bag. The sample in bag was stomached for 30 seconds to assist with removal of bacteria from meat. The stomached solution was ten-fold sequentially further diluted by transferring 1 mL into 9 mL the Letheen broth. Each diluted solution was analyzed using PETRIFILM AC and PETRIFILM EB as media. Plates with counts that are within the counting range of the plates (25-250 CFU per PETRIFILM AC plate and 15-100 CFU per PETRIFILM EB plate) were used for analysis. The results were converted to log 10 and the replicates averaged. The results of the treated meat samples were subtracted from the results of the analogous untreated meat samples to determine the log reduction of the treatment. Table 7 shows the data obtain from the tests described above.

| Table 7. | | | | | | | | | | | | |
|---|---|---|---|---|---|---|---|---|---|---|---|---|
| | Comp. Example 9 | | Example 5 | | Example 6 | | Example 7 | | | | Comp. Example 10 | |
| pH of additional enhancer Solution | 4.5 | | 5.5 | | 5.5 | | 5.5 (Test 1) | | 5.5 (Test 2) | | 5.5 | |
| Bacteria Test | AC | EB | AC | EB | AC | EB | AC | EB | AC | EB | AC | EB |
| Untreated meat native population | 2.62 | 0.49 | 5.29 | 3.16 | 2.62 | 0.49 | 5.29 | 3.16 | 7.14 | 1.90 | 7.14 | 1.90 |
| Treated meat population average | 3.01 | 0.33 | 4.57 | 3.34 | 2.36 | 0.33 | 0.93 | 0.000 | 4.49 | 0.77 | 5.80 | 2.07 |
| LOG Reduction | -0.39 | 0.16 | 0.72 | -0.17 | 0.26 | 0.16 | 4.37 | 3.16 | 2.65 | 1.13 | 1.34 | -0.17 |

The same experiments were repeated 3 times with different batches of meat and similar results were observed. The Examples in Table 7 indicate that at 4 °C on ground beef, three enhancers (carboxylic acid, bacteriocin, iron-binding protein) provide significant antimicrobial efficacy. This would be expected to extend the shelf-life of the meat without affecting sensory properties.

### Example 8 (comparative examples, not forming part of the invention)

Example 5 was repeated with the following noted differences. A pressurized sprayer (Sprayer Systems Co., Wheaton, IL) with a fan nozzle was used. The KITCHEN-AID mixer blehded the combination of Part A (undiluted) and Part B(containing both lactoferrin and nisin) with the paddle attachment for 3 minutes total at a low mix setting. The enhancer, Part B, was delivered first at a spray rate of 30 mL/min during the first 1.5 minutes of mixing. Part A, Concentrate 2, was not diluted in this case and was delivered at a spray rate of 7.5 mL/min while the mixing continued for an additional 1.5 minutes (min). Sufficient enhancer and antimicrobial lipid were added to deliver 1 wt-% of Concentrate 2 and 4 wt-% enhancer solution to the meat. The initial native bacteria was 40-80 counts/gram, detected with PETRIFILM AC. Ten minutes after treatment, the native bacteria became undetectable.

### Examples 9-28 (comparative examples, not forming part of the invention) and Comparative Examples C11-C15

Antimicrobial liquid compositions (Examples 9-28 and Comparative Examples C11-C15), having potential utility in the human oral cavity, were prepared by combining the components listed in Tables 8 and 9. Each liquid composition contained water (with 0.5 wt-% PLURONIC P65 surfactant) as a solvent system, two fatty acid monoesters (PGMC8 and GMLC12), an anionic surfactant (DOSS), optionally an organic acid enhancer (benzoic acid), and anenhancer selected from the group including sucrose, xylitol, lactoferrin, and nisin. Compositions without a secondenhancer (i.e., only an organic acid enhancer) were considered as Controls (designated with letter C).

| Table 8: Antimicrobial Compositions | | | | | | | | | | |
|---|---|---|---|---|---|---|---|---|---|---|
| Component (Grams) | Example | | | | | | | | | |
| | C11 | 9 | 10 | 11 | 12 | C12 | 13 | 14 | 15 | 16 |
| Water with 0.5% PLURONIC P65 | 100 | 100 | 100 | 100 | 100 | 100 | 100 | 100 | 100 | 100 |
| PGMC8 | 0.03 | 0.03 | 0.03 | 0.03 | 0.03 | 0.3 | 0.3 | 0.3 | 0.3 | 0.3 |
| GMLC12 | 0.03 | 0.03 | 0.03 | 0.03 | 0.03 | 0.3 | 0.3 | 0.3 | 0.3 | 0.3 |
| DOSS | 0.02 | 0.02 | 0.02 | 0.02 | 0.02 | 0.2 | 0.2 | 0.2 | 0.2 | 0.2 |
| Sucrose | | 1 | | | | | 1 | | | |
| Xylitol | | | 1 | | | | | 1 | | |
| Lactoferrin | | | | 1 | | | | | 1 | |
| Nisin | | | | | 0.15 | | | | | 0.15 |
| Wt-% (PGMC8 + GMLC12 + DOSS) | 0.08 | 0.08 | 0.08 | 0.08 | 0.08 | 0.8 | 0.8 | 0.8 | 0.8 | 0.8 |

| Table 9: Antimicrobial Compositions | | | | | | | | | | | | | | | |
|---|---|---|---|---|---|---|---|---|---|---|---|---|---|---|---|
| Component (Grams) | Example | | | | | | | | | | | | | | |
| | C13 | 17 | 18 | 19 | 20 | C14 | 21 | 22 | 23 | 24 | C15 | 25 | 26 | 27 | 28 |
| Water with 0.5% PLURONIC P65 | 100 | 100 | 100 | 100 | 100 | 100 | 100 | 100 | 100 | 100 | 100 | 100 | 100 | 100 | 100 |
| PGMC8 | 0.03 | 0.03 | 0.03 | 0.03 | 0.03 | 0.3 | 0.3 | 0.3 | 0.3 | 0.3 | 0.016 | 0.016 | 0.016 | 0.016 | 0.016 |
| GMLC12 | 0.03 | 0.03 | 0.03 | 0.03 | 0.03 | 0.3 | 0.3 | 0.3 | 0.3 | 0.3 | 0.016 | 0.016 | 0.016 | 0.016 | 0.016 |
| DOSS | 0.02 | 0.02 | 0.02 | 0.02 | 0.02 | 0.2 | 0.2 | 0.2 | 0.2 | 0.2 | 0.011 | 0.011 | 0.011 | 0.011 | 0.011 |
| Benzoic Acid | 0.03 | 0.03 | 0.03 | 0.03 | 0.03 | 0.3 | 0.3 | 0.3 | 0.3 | 0.3 | 0.016 | 0.016 | 0.016 | 0.016 | 0.016 |
| Sucrose | | 1 | | | | | 1 | | | | | 1 | | | |
| Xylitol | | | 1 | | | | | 1 | | | | | 1 | | |
| Lactoferrin | | | | 1 | | | | | 1 | | | | | 1 | |
| Nisin | | | | | 0.15 | | | | | 0.15 | | | | | 0.15 |
| Wt.-% (PGMC8 + GMLC12 + DOSS + Benzoic Acid) | 0.11 | 0.11 | 0.11 | 0.11 | 0.11 | 1.1 | 1.1 | 1.1 | 1.1 | 1.1 | 0.06 | 0.06 | 0.06 | 0.06 | 0.06 |

### Evaluations and Results

The antimicrobial compositions (Examples 9-28 and Comparative Examples C11-C 15) were evaluated for antibacterial activity according to the Antibacterial (*Streptococcus mutans*) Kill Rate Test Method as follows. Results are provided in Table 10.

### Antibacterial (Streptococcus mutans') Kill Rate Test Method.

A sample of 0.1 mL of *S. mutans* (ATCC # 25175) at 10⁸ CFU/ml in brain heart infusion (BHI) broth was mixed with 19.9 mL of liquid test antimicrobial sample at a given concentration in water for a predetermined time (0.5 minute, 2 minutes, 5 minutes, and 10 minutes each). Immediately after mixing for the predetermined time, 1.0 mL of the sample was transferred from the flask into a test tube containing 9.0 mL Letheen Broth (VWR Scientific, Batavia, IL) to neutralize for the fatty acid monoester and benzoic acid components that might be present in the sample. A Vortex mixer was used for thorough mixing and the resulting solution was designated as the 10"¹ dilution. A 1.0-mL aliquot was transferred from the 10⁻¹ dilution into a second tube containing 9.0 mL Letheen Broth and mixed as above to give a solution designated the 10"² dilution. Aliquots (0.1 mL) from each of the 10⁻¹ and 10⁻² dilutions were plated out in duplicate and spread on sheep blood agar with hockey-stick applicators on Petri dish plates to provide 10⁻² and 10⁻³ concentrations on each respective plate. The Petri dishes were incubated for 96 hours at 37°C aerobically followed by counting the number of colony forming Units (CFU). This Information was used to compute kill rates for *S. mutans* at a specified concentration of test sample.

| Table 10: Results of Kill Rate Testing against *Streptococcus mutans* | | | | | | | | |
|---|---|---|---|---|---|---|---|---|
| Example | Organic Acid Enhancer | Enhancer | Additive Wt.-% (PGMC8 + GMLC12 + DOSS + Benzoic Acid) | Initial - Log (Bacteria 1 Count) | Reduction - Log (Bacterial Count) | | | |
| | | | | | 0.5 Min. | 2 Min. | 5 Min. | 10 Min. |
| C11 | None | None (Control) | 0.08 | 6.8 | 1.26 | 1.26 | 1.26 | 2.83 |
| C12 | None | None (Control) | 0.8 | 6.8 | 1.26 | 1.26 | 4.8 | 4.8 |
| 9 | None | 1% Sucrose | 0.08 | 6.8 | 1.26 | 1.26 | 1.26 | 1.26 |
| 13 | None | 1% Sucrose | 0.8 | 6.8 | 1.97 | 3.71 | 4.8 | 4.8 |
| 10 | None | 1% Xylitol | 0.08 | 6.8 | 1.26 | 1.26 | 1.26 | 1.8 |
| 14 | None | 1% Xylitol | 0.8 | 6.8 | 2.38 | 2.87 | 3.78 | 4.8 |
| 11 | None | 1% Lactoferrin | 0.08 | 6.8 | 2.16 | 2.36 | 2.49 | 1.88 |
| 15 | None | 1 % Lactoferrin | 0.8 | 6.8 | 1.26 | 1.81 | 1.77 | 2.01 |
| 12 | None | 0.15% Nisin | 0.08 | 6.08 | 4.08 | 4.08 | 4.08 | 4.08 |
| 16 | None | 0.15% Nisin | 0.8 | 6.08 | 4.08 | 4.08 | 4.08 | 4.08 |
| C15 | Benzoic Acid | None (Control) | 0.06 | 6.65 | 2.66 | 4.65 | 4.65 | 4.65 |
| C13 | Benzoic Acid | None (Control) | 0.11 | 6.8 | 4.39 | 4.8 | 4.8 | 4.8 |
| C14 | Benzoic Acid | None (Control) | 1.1 | 6.8 | 4.8 | 4.8 | 4.8 | 4.8 |
| 25 | Benzoic Acid | 1% Sucrose | 0.06 | 6.65 | 2.73 | 4.65 | 4.65 | 4.65 |
| 17 | Benzoic Acid | 1% Sucrose | 0.11 | 6.8 | 4.38 | 4.07 | 4.8 | 4.52 |
| 21 | Benzoic Acid | 1% Sucrose | 1.1 | 6.8 | 4.39 | 4.8 | 4.8 | 4.8 |
| 26 | Benzoic Acid | 1% Xylitol | 0.06 | 6.65 | 2.55 | 4.65 | 4.65 | 4.65 |
| 18 | Benzoic Acid | 1% Xylitol | 0.11 | 6.8 | 4.68 | 4.68 | 4.8 | 4.8 |
| 22 | Benzoic Acid | 1% Xylitol | 1.1 | 6.8 | 4.8 | 4.8 | 4.8 | 4.8 |
| 27 | Benzoic Acid | 1% Lactoferrin | 0.06 | 6.65 | 1.1 | 1.1 | 1.1 | 1.1 |
| 19 | Benzoic Acid | 1% Lactoferrin | 0.11 | 6.08 | 4.08 | 4.08 | 4.08 | 4.08 |
| 23 | Benzoic Acid | 1% Lactoferrin | 1.1 | 6.08 | 4.02 | 4.08 | 4.08 | 4.08 |
| 28 | Benzoic Acid | 0.15% Nisin | 0.06 | 6.65 | 1.1 | 3.42 | 4.65 | 4.65 |
| 20 | Benzoic Acid | 0.15% Nisin | 0.11 | 6.08 | 4.08 | 4.08 | 4.08 | 4.08 |
| 24 | Benzoic Acid | 0.15% Nisin | 1.1 | 6.08 | 4.08 | 4.08 | 4.08 | 4.08 |

It can be concluded from the data in Table 10 that, in general, the antimicrobial compositions that contained the organic acid enhancer (benzoic acid) and the antimicrobial compositions that contained both organic acid enhancer and enhancer showed the greatest levels of *S. mutans* log reduction. It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. An antimicrobial composition, comprising:
an antimicrobial lipid component comprising a compound selected from the group consisting of a fatty ether of a polyhydric alcohol, alkoxylated derivatives thereof, and combinations thereof; and an enhancer component comprising:
a compound selected from the group consisting of iron-binding proteins and derivatives thereof, siderophores, and combinations thereof.

2. An antimicrobial composition, comprising:
an antimicrobial lipid component comprising a compound selected from the group consisting of a saturated fatty ether of a polyhydric alcohol, an unsaturated fatty ether of a polyhydric alcohol, alkoxylated derivatives thereof, and combinations thereof, wherein the alkoxylated derivative has less than 5 moles of alkoxide per mole of polyhydric alcohol; with the proviso that the antimicrobial lipid does not include a glycerol monoester; and
an enhancer component comprising a compound selected from the group consisting of iron-binding proteins and derivatives thereof, siderophores, and combinations thereof.

3. The composition of claim 1 or claim 2 further comprising a surfactant.

4. The composition of any one of claims 1 through 3 wherein the enhancer component comprises nisin and lactoferrin or derivatives thereof.

5. The composition of any one of claims 1 through 3 wherein the enhancer component comprises an organic acid, lactoferrin, and either a sugar, a sugar alcohol, or both.

6. The composition of any one of claims 1 through 5 which demonstrates at least a one-log average reduction of total aerobic bacteria count.

7. The composition of any one of claims 1 through 6 wherein the antimicrobial lipid component is present in the composition in a major amount.

8. The composition of any one of claims 1 through 7 in the form of a dental composition.

9. The composition of claim 8 which is in the form of a dental product selected from the group consisting of an oral rinse, an oral irrigational solution, a remineralization solution, a prophy paste, a prophy powder, a sub-gingival cleanser, a dentrifrice, a denture adhesive, and an etchant.

10. The composition of claim 8 further comprising an antimicrobial enzyme.

11. An antimicrobial kit comprising the antimicrobial composition of any one of claims 1 through 10, wherein the kit comprises a first container comprising the antimicrobial lipid component, and a second container comprising the enhancer component.

12. A method of using the composition of any one of claims 1 through 10, the method comprising applying the composition to a surface with the proviso applying the composition does not constitute treatment of a human or animal body.

13. The method of claim 12 wherein the surface is the surface of a substrate selected from the group consisting of meat, meat products, plants, and plant parts.

14. The method of claim 12 wherein the surface is the surface of an inanimate substrate selected from the group consisting of textiles, glass, polymeric surfaces, metal, wood, and rubber.

15. The method of claim 12 wherein the surface is the surface of skin or hair of a mammal.

16. The method of claim 12 wherein the surface is the surface of a dental product.

17. The method of claim 16 wherein the dental product is a dental impression tray, a dental instrument, dental floss, dental pick, dental tape, or dental packing.

18. The method of any one of claims 12 through 17 further comprising diluting the composition with a vehicle before applying the composition to a substrate.

19. A method of applying an antimicrobial composition to a substrate, the method comprising applying to the substrate a major amount of an antimicrobial lipid component comprising a compound selected from the group consisting of a fatty ether of a polyhydric alcohol, alkoxylated derivatives thereof, and combinations thereof; and
applying to the substrate a minor amount of an enhancer component comprising a compound selected from the group consisting of iron-binding proteins and derivatives thereof, siderophores, and combinations thereof.

20. The method of claim 19 wherein the antimicrobial lipid component is applied to the Substrate before the enhancer component is applied to the substrate or the antimicrobial lipid component is applied to the substrate after the enhancer component is applied to the substrate.

21. A composition according to claims 1 to 10 for use in the prevention or treatment of dental caries, periodontal disease, or a carious lesion.

## Patentansprüche

1. Antimikrobielle Zusammensetzung, die Folgendes umfasst:
eine antimikrobielle Lipidkomponente, die eine Verbindung ausgewählt aus der Gruppe bestehend aus einem Fettether eines mehrwertigen Alkohols, alkoxylierten Derivaten davon, und Kombinationen davon umfasst; und eine Enhancer-Komponente, die Folgendes umfasst:
eine Verbindung ausgewählt aus der Gruppe bestehend aus eisenbindenden Proteinen und Derivaten davon, Siderophoren, und Kombinationen davon.

2. Antimikrobielle Zusammensetzung, die Folgendes umfasst:
eine antimikrobielle Lipidkomponente, die eine Verbindung ausgewählt aus der Gruppe bestehend aus einem gesättigten Fettether eines mehrwertigen Alkohols, einem ungesättigten Fettether eines mehrwertigen Alkohols, alkoxylierten Derivaten davon, und Kombinationen davon umfasst, wobei das alkoxylierte Derivat weniger als 5 mol Alkoxid pro mol an mehrwertigem Alkohol aufweist; mit der Maßgabe, dass das antimikrobielle Lipid keinen Glycerinmonoester umfasst; und
eine Enhancer-Komponente, die eine Verbindung ausgewählt aus der Gruppe bestehend aus eisenbindenden Proteinen und Derivaten davon, Siderophoren, und Kombinationen davon umfasst.

3. Zusammensetzung nach Anspruch 1 oder Anspruch 2, die ferner ein grenzflächenaktives Mittel umfasst.

4. Zusammensetzung nach einem der Ansprüche 1 bis 3, wobei die Enhancer-Komponente Nisin und Lactoferrin oder Derivate davon umfasst.

5. Zusammensetzung nach einem der Ansprüche 1 bis 3, wobei die Verbesserungskomponente eine organische Säure, Lactoferrin und entweder einen Zucker, einen Zuckeralkohol oder beides umfasst.

6. Zusammensetzung nach einem der Ansprüche 1 bis 5, die wenigstens eine durchschnittliche Verringerung um 1 log-Stufe der Gesamtbakterienzahl zeigt.

7. Zusammensetzung nach einem der Ansprüche 1 bis 6, wobei die antimikrobielle Lipidkomponente in der Zusammensetzung in einer größeren Menge vorliegt.

8. Zusammensetzung nach einem der Ansprüche 1 bis 7 in Form einer Dentalzusammensetzung.

9. Zusammensetzung nach Anspruch 8, die in Form eines Dentalproduktes vorliegt, das ausgewählt ist aus der Gruppe bestehend aus einer Mundspülung, einer Mundduschenlösung, einer Remineralisierungslösung, einer Prophylaxepaste, einem Prophylaxepuder, einem subgingivalen Reinigungsmittel, einem Zahnputzmittel, einem Zahnprothesenklebstoff und einem Ätzmittel.

10. Zusammensetzung nach Anspruch 8, die ferner ein antimikrobielles Enzym umfasst.

11. Antimikrobielles Set, das die antimikrobielle Zusammensetzung nach einem der Ansprüche 1 bis 10 umfasst, wobei das Set einen ersten Behälter, der die antimikrobielle Lipidkomponente umfasst, und einen zweiten Behälter, der die Enhancer-Komponente umfasst, umfasst.

12. Verfahren zur Verwendung der Zusammensetzung nach einem der Ansprüche 1 bis 10, wobei das Verfahren das Aufbringen der Zusammensetzung auf eine Oberfläche umfasst, mit der Maßgabe, dass das Aufbringen der Zusammensetzung nicht die Behandlung eines menschlichen oder tierischen Körpers darstellt.

13. Verfahren nach Anspruch 12, wobei die Oberfläche die Oberfläche eines Substrats ist, das ausgewählt ist aus der Gruppe bestehend aus Fleisch, Fleischprodukten, Pflanzen und Pflanzenteilen.

14. Verfahren nach Anspruch 12, wobei die Oberfläche die Oberfläche eines unbelebten Substrats ist, ausgewählt aus der Gruppe aus Textilien, Glas, polymeren Oberflächen, Metall, Holz und Gummi.

15. Verfahren nach Anspruch 12, wobei die Oberfläche die Oberfläche von Haut oder Haar eines Säugetiers ist.

16. Verfahren nach Anspruch 12, wobei die Oberfläche die Oberfläche eines Dentalproduktes ist.

17. Verfahren nach Anspruch 16, wobei das Dentalprodukt eine dentale Abdruckplatte, ein Dentalinstrument, Zahnseide, Dentalkürette, Zahnreinigungsband oder Dentalverpackung ist.

18. Verfahren nach einem der Ansprüche 12 bis 17, das ferner das Verdünnen der Zusammensetzung mit einem Vehikel vor dem Aufbringen der Zusammensetzung auf ein Substrat umfasst.

19. Verfahren zum Anwenden einer einer antimikrobiellen Zusammensetzung auf einem Substrat, wobei das Verfahren Folgendes umfasst:
das Aufbringen einer größeren Menge einer antimikrobiellen Lipidkomponente, die eine Verbindung umfasst, die ausgewählt ist aus der Gruppe bestehend aus einem Fettether eines mehrwertigen Alkohols, alkoxylierten Derivaten davon, und Kombinationen davon, auf das Substrat; und
das Aufbringen einer kleineren Menge einer Enhancer-Komponente, die eine Verbindung ausgewählt aus der Gruppe bestehend aus ionenbindenden Proteinen und Derivaten davon, Siderophoren und Kombinationen davon umfasst, auf das Substrat.

20. Verfahren nach Anspruch 19, wobei die antimikrobielle Lipidkomponente auf das Substrat aufgebracht wird, bevor die Verbesserungskomponente aufgebracht wird, oder die antimikrobielle Lipidkomponente auf das Substrat aufgebracht wird, nachdem die Enhancer-Komponente auf das Substrat aufgebracht wird.

21. Zusammensetzung nach den Ansprüchen 1 bis 10 zur Verwendung bei der Prävention oder Behandlung von Zahnkaries, Periodontalerkrankung oder einer kariösen Verletzung.

## Revendications

1. Composition antimicrobienne, comprenant :
un composant lipidique antimicrobien comprenant un composé choisi dans le groupe constitué d'un éther gras d'un alcool polyhydrique, des dérivés alcoxylés de celui-ci, et leurs combinaisons ; et un composant adjuvant comprenant :
un composé choisi dans le groupe constitué de protéines de liaison de fer et leurs dérivés, des sidérophores, et leurs combinaisons.

2. Composition antimicrobienne, comprenant :
un composant lipidique antimicrobien comprenant un composé choisi dans le groupe constitué d'un éther gras saturé d'un alcool polyhydrique, un éther gras insaturé d'un alcool polyhydrique, des dérivés alcoxylés de ceux-ci, et leurs combinaisons, dans laquelle le dérivé alcoxylé possède moins de 5 moles d'alcoxyde par mole d'alcool polyhydrique ; à condition que le lipide antimicrobien n'inclue pas de monoester de glycérol ; et
un composant adjuvant comprenant un composé choisi dans le groupe constitué de protéines de liaison de fer et leurs dérivés, des sidérophores, et leurs combinaisons.

3. Composition selon la revendication 1 ou la revendication 2, comprenant en outre un agent tensioactif.

4. Composition selon l'une quelconque des revendications 1 à 3, dans laquelle le composant adjuvant comprend de la nisine et de la lactoferrine ou leurs dérivés.

5. Composition selon l'une quelconque des revendications 1 à 3, dans laquelle le composant adjuvant comprend un acide organique, de la lactoferrine, et soit un sucre, soit un alcool glucidique, soit l'un et l'autre.

6. Composition selon l'une quelconque des revendications 1 à 5, qui démontre au moins une réduction moyenne d'un logarithme de la numération totale de bactéries aérobies.

7. Composition selon l'une quelconque des revendications 1 à 6, dans laquelle le composant lipidique antimicrobien est présent dans la composition en une quantité majeure.

8. Composition selon l'une quelconque des revendications 1 à 7, sous la forme d'une composition dentaire.

9. Composition selon la revendication 8, qui est sous la forme d'un produit dentaire choisi dans le groupe constitué d'un produit de rinçage oral, une solution d'irrigation orale, une solution de reminéralisation, une pâte prophylactique, une poudre prophylactique, un nettoyant sous-gingival, un dentifrice, un adhésif pour dentier, et un décapant.

10. Composition selon la revendication 8, comprenant en outre une enzyme antimicrobienne.

11. Trousse antimicrobienne comprenant la composition antimicrobienne selon l'une quelconque des revendications 1 à 10, où la trousse comprend un premier récipient comprenant le composant lipidique antimicrobien, et un deuxième récipient comprenant le composant adjuvant.

12. Procédé d'utilisation de la composition selon l'une quelconque des revendications 1 à 10, le procédé comprenant l'application de la composition sur une surface à condition que l'application de la composition ne constitue pas un traitement d'un organisme humain ou animal.

13. Procédé selon la revendication 12, dans lequel la surface est la surface d'un substrat choisi dans le groupe constitué de viande, produits de viande, plantes, et parties de plante.

14. Procédé selon la revendication 12, dans lequel la surface est la surface d'un substrat inanimé choisi dans le groupe constitué de textiles, verre, surfaces polymères, métal, bois, et caoutchouc.

15. Procédé selon la revendication 12, dans lequel la surface est la surface de la peau ou des poils d'un mammifère.

16. Procédé selon la revendication 12, dans lequel la surface est la surface d'un produit dentaire.

17. Procédé selon la revendication 16, dans lequel le produit dentaire est un porte-empreinte dentaire, un instrument dentaire, du fil dentaire, une pointe dentaire, un ruban dentaire, ou un tampon dentaire.

18. Procédé selon l'une quelconque des revendications 12 à 17, comprenant en outre la dilution de la composition avec un véhicule avant application de la composition sur un substrat.

19. Procédé d'application d'une composition antimicrobienne sur un substrat, le procédé comprenant l'application sur le substrat d'une quantité majeure d'un composant lipidique antimicrobien comprenant un composé choisi dans le groupe constitué d'un éther gras d'un alcool polyhydrique, des dérivés alcoxylés de celui-ci, et leurs combinaisons ; et
l'application sur le substrat d'une quantité mineure d'un composant adjuvant comprenant un composant adjuvant comprenant un composé choisi dans le groupe constitué de protéines de liaison de fer et leurs dérivés, des sidérophores, et leurs combinaisons.

20. Procédé selon la revendication 19, dans lequel le composant lipidique antimicrobien est appliqué sur le substrat avant que le composant adjuvant soit appliqué sur le substrat ou le composant lipidique antimicrobien est appliqué sur le substrat après que le composant adjuvant est appliqué sur le substrat.

21. Composition selon les revendications 1 à 10, à utiliser dans la prévention ou le traitement de caries dentaires, de maladie périodontique, ou d'une lésion cariée.
